(19) Europäisches Patentamt
European Patent Office
Office européen des brevets

(11) Veröffentlichungsnummer: **0 194 541 B1**

(12) # EUROPÄISCHE PATENTSCHRIFT

(45) Veröffentlichungstag der Patentschrift: **21.11.91**

(51) Int. Cl.⁵: **C07C 51/377**, C07C 57/04, B01J 27/198, C01B 25/16, C01G 39/00

(21) Anmeldenummer: **86102753.0**

(22) Anmeldetag: **03.03.86**

(54) **Heteropolysäure H8PMo10VO39, deren Anhydrid PMo10VO35 und ihre Verwendung.**

(30) Priorität: **12.03.85 DE 3508649**

(43) Veröffentlichungstag der Anmeldung:
**17.09.86 Patentblatt 86/38**

(45) Bekanntmachung des Hinweises auf die Patenterteilung:
**21.11.91 Patentblatt 91/47**

(84) Benannte Vertragsstaaten:
**BE DE FR GB IT NL**

(56) Entgegenhaltungen:
**EP-A- 0 046 840**
**EP-A- 0 064 371**
**EP-A- 0 113 084**
**DE-A- 2 722 375**
**DE-A- 3 010 434**
**DE-B- 2 704 991**
**US-A- 4 192 951**

(73) Patentinhaber: **Röhm GmbH**
**Kirschenallee Postfach 4242**
**W-6100 Darmstadt 1(DE)**

(72) Erfinder: **Gruber, Wilhelm, Dr.**
**Peter-Behrens-Strasse 34**
**W-6100 Darmstadt(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Gebiet der Erfindung

Die Erfindung betrifft eine neue Heteropolysäure mit Phosphor als Zentralatom und Molybdän sowie Vanadium als peripheren Atomen im Anion, Verfahren zu ihrer Herstellung sowie ihre Verwendung z.B. in Katalysatoren.

Stand der Technik

Eine Klassifizierung der Heteropolysäure des Molybdäns und Wolframs von G.A. Tsigdinos, Topics in Current Chemistry, 76, 1-64, (1978) zeigt, daß zu dieser Verbindungsklasse Verbindungen mit ganz verschiedenen Elementen als Zentralatome, in verschiedenen Atomverhältnissen und mit verschiedenen, z.T. noch unbekannten Kristallstrukturen, gehören. Zu einer dieser Serien, den 12-Heteropolysäuren der allgemeinen Formel

$H^{(8-n)}[X^{n+} Mo_{12} (W)_{12} O_{40}]$.

wobei X das Zentralatom bedeutet, gehören die mit am längsten bekannten und gut untersuchten Molybdän- und Wolframheteropolysäuren $H_3PMo_{12}O_{40} \cdot 30\ H_2O$ und $H_3PW_{12}O_{40} \cdot 29\ H_2O$. Eine andere Serie von Heteropolysäureverbindungen ist gekennzeichnet durch das Anion $[X^{n+}Mo_{11}O_{39}]^{-(12-n)}$, wobei $P^{5+}$, $As^{5+}$ und $Ge^{4+}$ als Zentralatome X enthalten sein können, wovon bisher nur das 11-Molybdogermanatanion bestimmt bekannt ist. Ihre Kristallstruktur ist noch unbekannt und nicht mit derjenigen welche die oben genannten 12-Heteropolysäuren haben, nämlich der nach ihrem Entdecker genannten Keggin-Struktur, identisch.

12-Molybdophosphorsäure, $H_3PMo_{12}O_{40}$, und deren Vanadiumderivate, d.h. Molybdovanadophosphorsäuren, $H_{3+x}PMo_{12-x}V_xO_{40}$, sind als Heteropolysäurenkatalysatoren für die Durchführung selektiver Oxidationen, so auch für die Oxidehydrierung von Isobuttersäure oder ihren Estern zu Methacrylsäure oder ihren Estern, bekannt. In Studies Surfactant Science and Catalysis, 7 (1981), 780-791, ist gezeigt, daß der teilweise Ersatz von Molybdän durch Vanadium, zu $H_5PMo_{10}V_2O_{40}$, die Methacrylsäureselektivität des $H_3PMo_{12}O_{40}$-Katalysators von 40 % auf 73 %, bei fast gleichbleibendem Isobuttersäureumsatz, erhöht.

Die Herstellung der Molybdovanadophosphorsäuren $H_4PMo_{11}VO_{40}$,$H_5\ PMo_{10}V_2O_{40}$ und $H_6PMo_9V_3O_{40}$ beschreiben Tsigdinos und Fallada in Inorg.Chem. 7 (1968), S. 437-441, und ein weiteres Verfahren, das im Gegensatz zu dem vorerwähnten Verfahren nicht von Salzen der Ausgangsverbindungen, sondern von den Oxiden bzw. den freien Säuren, wie $MoO_3$, $V_2O_5$ und $H_3PO_4$ ausgeht, ist in DE-OS 27 22 375 angegeben. Hier wird in Beispielen praktisch nur die Herstellung der Heteropolysäure $H_5PMo_{10}V_2O_{40}$ angeführt, die anschließend für die Herstellung von Oxidationskatalysatoren weiterverwendet wird, mit denen dann verschiedene Oxidationsreaktionen, u.a. die Oxidehydrierung von Isobuttersäure und deren Methylester zu Methacrylsäure und deren Methylester durchgeführt werden.

In der DE-OS 30 10 434 wird ein Verfahren zur Herstellung von Methacrylsäure durch Oxidation von Methacrolein angegeben, das in Gegenwart von Katalysatoren der allgemeinen Formel $Mo_aV_bP_cX_dY_eO_g$ abläuft, worin X ein oder mehrere Elemente, ausgewählt aus der Gruppe Kupfer, Zinn, Thorium, Germanium, Nickel, Eisen, Kobalt, Zink, Titan, Blei, Aluminium, Zirkonium, Cer, Wismut und Arsen, Y ein oder mehrere Elemente aus der Gruppe Kalium, Rubidium, Cäsium und Thallium sind und a, b, c, d, e und f die Atomverhältnisse bedeuten. Diese Katalysatoren werden in der DE-OS als Katalysatoren mit Heteropolysäuresalzstruktur bezeichnet. Beispiel eines solchen Katalysators ist das Produkt mit der Zusammensetzung $Mo_{10}V_1P_1Cu_{0.2}$ und der äquivalenten Sauerstoffmenge.

Die DE-OS 32 48 600 beschreibt, daß Mo-V-Cu-P-Katalysatoren, z.B. Katalysatoren mit $Cu_{0.2}PMo_{10}VO_{35,2}$, die Oxidehydrierung von Isobuttersäure zu Methacrylsäure in hohen Selektivitäten, bis 82,7 %, beschleunigen. Diese Katalysatoren haben jedoch den großen Nachteil, daß sie relativ schnell desaktiviert werden und dadurch eine technisch gesehen zu kurze Lebensdauer haben.

Der Temperaturbereich für die Durchführung der oxidativen Dehydrierung von Isobuttersäure bzw. deren Estern mit Heteropolysäurekatalysatoren ist 250 bis 500°C, bevorzugt 300 bis 400°C. Mit Hilfe der IR-Spektroskopie wurde an Katalysatorproben aus $H_5PMo_{10}V_2O_{40}$ durch das Auftreten von $MoO_3$-Banden ermittelt, daß der hier vorliegende "Heteropolysäureverband" bei 375°C deutlich in seine oxidischen Bestandteile zerfällt. Dieser thermische Zerfall in dem für die oxidative Dehydrierung wichtigen Temperaturgebiet ist ein Hauptfaktor für die stetige Abnahme der Aktivitäten der Katalysatoren aus $H_5PMo_{10}\ V_2O_{40}$ und $Cu_{0,2}\ PMo_{10}VO_{35,2}$.

Aufgabe und Lösung

Es war daher notwendig nach einer thermisch stabileren Heteropolysäure mit den Elementen Phosphor, Molybdän und Vanadium zu suchen, die auch in chemischen Reaktionen als Katalysator brauchbar ist.
Überraschenderweise wurde gefunden, daß bei der Umsetzung von Molybdäntrioxid und Vanadiumpentoxid in wäßriger Phosphorsäure im Molverhältnis 10 : 0,5 : 1 nach dem in der DE-OS 27 22 375 beschriebenen hydrothermischen Umsetzungsverfahren, in praktisch quantitativer Ausbeute eine neue Heteropolysäure, die 10-Molybdovanadophosphorsäure, der Formel

$H_8PMo_{10}VO_{39}$

entsteht. Sie ist eine Spezies der weiter oben angegebenen und wenig bekannten $[X^{n+}Mo_{11}O_{39}]^{-(12-n)}$-Serie mit $P^{5+}$ als Zentralatom $X^{n+}$ und der Substitution von einem Mo-Atom durch ein V-Atom, wodurch sich die 8-fach negative Ladung des $PMo_{10}VO_{39}$-Anions ergibt. Aus der neuen Heteropolysäure laßt sich durch Dehydratisierung ihr Anhydrid, die neue Verbindung $PMo_{10}VO_{35}$, herstellen.
Weiter wurde gefunden, daß die neue Heteropolysäure und ihr Anhydrid gute katalytische Eigenschaften, insbesondere bei Oxidations-/Dehydrierungsvorgängen, besitzen. So wurde z.B. gefunden, daß die Nachteile der bisherigen Verfahren zur oxidativen Dehydrierung von Isobuttersäure zu Methacrylsäure, nämlich die rasche Desaktivierung der Katalysatoren, mit Katalysatoren aus der neuen Molybdovanadophosphorsäure überwunden werden.
Gegenstand der vorliegenden Erfindung ist somit die neue Heteropolysäure $H_8PMo_{10}VO_{39}$, ihr Anhydrid $PMo_{10}VO_{35}$, Verfahren zu ihrer Herstellung und Verwendung der neuen Stoffe als Katalysatoren.

Eigenschaften der neuen Heteropolysäure

Die neue Verbindung fällt bei der Kristallisation aus wäßrigem Medium in orangefarbenen Kristallen als kristallwasserhaltige Verbindung mit etwa 25 bis 35 Molen Kristallwasser, meist als

$H_8PMo_{10}VO_{39} . 32 H_2O$

an und liegt in sehr reiner Form vor.
Ihr thermisches Verhalten laßt sich durch das folgende Schema wiedergeben;

$$H_8PMo_{10}VO_{39} \cdot 32H_2O \xrightarrow[-32H_2O]{100 - 200°C} H_8PMo_{10}VO_{39}$$

$$H_8PMo_{10}VO_{39} \xrightarrow[-4H_2O]{200 - 400°C} PMo_{10}VO_{35}$$

$$PMo_{10}VO_{35} \xrightarrow{\text{ab ca. } 425°C} MoO_3 + \ldots$$

Die orangefarbene kristallwasserhaltige Verbindung nimmt nach Abgabe des Kristallwassers einen orange-bräunlichen Farbton an, der bei der Abgabe des Konstitutionswassers durch Bildung von $PMo_{10}VO_{35}$ ockerfarben wird. Durch Behandeln mit Wasser läßt sich aus dem Anhydrid die Heteropolysäure zurückbilden. Aus IR-Untersuchungen getemperter Proben läßt sich eine deutlich höhere thermische Stabilität des P-Mo-V-O-Verbandes der $H_8PMo_{10}VO_{39}$-Heteropolysäure bzw. ihres Anhydrides als desjenigen der $H_5PMo_{10}V_2O_{40}$-Heteropolysäure ersehen:

| | Beginn der Zersetzung ab | vollst. $MoO_3$-Spektrum bis |
|---|---|---|
| $H_5PMo_{10}V_2O_{40}$ | ca. 375°C | 400°C |
| $H_8PMo_{10}VO_{39}$ | ca. 425°C | 450°C |

Nach $^{31}$P-NMR-Untersuchungen zeigt $H_8PMo_{10}VO_{39}$ nur ein Signal bei $\delta$ = -3.35 ppm mit $H_3PO_4$ als externen Standard, während bei der Untersuchung auch von mehrfach umkristallisierter $H_5PMo_{10}V_2O_{40}$ mehrere Signale, mit $\delta$: -3,89, -3,62 und -3,53 ppm, auftreten. Diese Ergebnisse zeigen, daß die neue Heteropolysäure in reiner Form erhalten wird und bestätigen die Aussagen sowohl von Tsigdinos und Fallada, Inorg. Chem. 7 (1968), S. 437-441, als auch von Spitsyn et al., Sov.Sci.Rev., Sect. B 1981, 3, S. 135-140, daß $H_5PMo_{10}V_2O_{40}$ sowohl ein Gemisch von isomeren Verbindungen, als auch mit den Heteropolysäuren $H_4PMo_{11}VO_{40}$ und $H_6PMo_9V_3O_{40}$ verunreinigt, sein kann. Nach Spitsyn et al. gibt nur die NMR-Methode die Möglichkeit die Individualität des Heteropolysäureanions aufzuzeigen.

$H_8PMo_{10}VO_{39}$ hat als typische Heteropolysäure stark saure und oxidierende Eigenschaften. Als oxidierendes Agens wird sie reduziert, kann aber wieder leicht reoxidiert werden. Infolge dieser ausgeprägten Eigenschaften lassen sich die neue Heteropolysäure und das aus ihr herstellbare Anhydrid in hervorragender Weise in homogener und heterogener Arbeitsweise als Katalysatoren bei vielen Reaktionen verwenden. Ihre sauren Eigenschaften geben Katalysatoren z.B. für Veresterungen oder für Additionen von H-aktiven Verbindungen an Mehrfachbindungen wie z.B. die Addition von $H_2O$ oder Alkoholen an Olefine. Durch ihre oxidierenden Eigenschaften und ihrer schnellen Reoxidation, lassen sich $H_8PMo_{10}VO_{39}$ und $PMo_{10}VO_{35}$ als Katalysatoren in homogener und heterogener Phase bei Oxidationen, Dehydrierungen und oxidativen Dehydrierungen einsetzen. Beispiele solcher Reaktionen sind Oxidationen von Olefinen wie Propylen oder Isobutylen bzw. dessen Derivat tert.-Butanol zu den entsprechenden Aldehydehyden und deren Weiteroxidation zu den ungesättigten Säuren, wie Acrylsäure oder Methacrylsäure. Weitere Beispiele sind die oxidative Dehydrierung von Äthylbenzol zu Styrol oder diejenige von Isobuttersäure oder ihrer Ester zu Methacrylsäure oder ihren Estern. Auch die Ammonoxidation zu gesättigten oder ungesättigten Nitrilen und die Herstellung von Maleinsäureanhydrid aus Benzol oder $C_4$-Kohlenwasserstoffen wird durch die neue Heteropolysäure oder ihr Anhydrid katalysiert.

Als eine weitere Einsatzmöglichkeit der neuen Heteropolysäure sei noch ihre Verwendung zur Herstellung von Farbstoffen genannt.

Vorteile der mit $H_8PMo_{10}VO_{39}$-Heteropolysäure hergestellten Katalysatoren bei der Oxidehydrierung von Isobuttersäure und ihren Estern

Wie durch Versuche mit aus der neuen Heteropolysäure hergestellten Katalysatoren gefunden wurde, stellen diese aktive und selektive Katalysatoren für die Oxidehydrierungsreaktion dar. Die höchsten Methacrylsäureselektivitäten, die bisher mit Katalysatoren aus der neuen Heteropolysäure erhalten wurden, liegen um 76 %. Sie liegen damit praktisch gleich hoch wie sie an mit $H_5PMo_{10}V_2O_{40}$ hergestellten Katalysatoren erreicht werden können und um wenige Prozentpunkte niedriger als nach dem in der DE-OS 32 48 600 beschriebenen Verfahren. Es wurde jedoch auch weiter gefunden, daß die neuen Katalysatoren durch die weiter oben besprochenen charakteristischen thermischen Eigenschaften der $H_8PMo_{10}VO_{39}$-Heteropolysäure auch thermisch stabiler sind als die bisher bekannten Heteropolysäure- und Heteropolysäuresalzkatalysatoren und damit eine wesentlich längere Lebensdauer bei der Oxidehydrierungsreaktion haben. Die in den Reaktoren mit den Katalysatoren gemessenen Temperaturen liegen wie aus den bekannten Beispielen hervorgeht, niedriger als z.B. die weiter oben für den beginnenden Zerfall der $H_5PMo_{10}V_2O_{40}$-Heteropolysäure angegebene Temperatur von 375°C. Solche Reaktionstemperaturangaben sind makroskopische Größen, die nichts über die am Ort der katalytischen Oxidation auftretenden Temperaturen aussagen. Hier können, wie bekannt, wesentliche Übertemperaturen herrschen, die sich zu meßbaren "hot spots" entwickeln können. Kommt man mit den Übertemperaturen und den hot spots in einen Temperaturbereich, in dem die katalytisch wirkende Substanz einer Phasenänderung unterliegt, kann sich das katalytische Verhalten ändern, was oft und wie auch im Falle der Heteropolysäuren mit einem Absinken der Lebensdauer und damit der Produktionsleistung einhergeht. Daher ist es immer günstig zwischen eingestellter Reaktionstemperatur und einer Phasenänderungstemperatur eine größere Differenz zu haben.

Dies wird mit der neuen, für die Oxidehydrierung aktiven und selektiven Heteropolysäure, erreicht.

Ausführung der Erfindung

a) Herstellung von $H_8PMo_{10}VO_{39} \cdot 32\ H_2O$

Die erfindungsgemäße neue Heteropolysäure wird nach dem in der DE-OS 27 22 375 zur Herstellung von Molybdo- bzw. wolframato-vanadophosphorsäuren angegebenen hydrothermischen Umsetzungsverfahren, d.h. durch Umsetzung der Oxide oder Oxysäuren von $Mo^{6+}$, $V^{5+}$ und $P^{5+}$ in wäßriger Phase bei Temperaturen von 60° oder mehr während einer meist mehrstündigen Reaktionszeit gebildet. Zur Entstehung der Heteropolysäure $H_8PMo_{10}VO_{39}$ werden die Ausgangskomponenten in einem solchen Verhältnis eingesetzt, daß die Stöchiometrie P : Mo : V = 1 : 10 : 1 eingestellt ist, was z.B. bei den Ausgangsstoffen $H_3PO_4$, $MoO_3$ und $V_2O_5$ eine Stöchiometrie von 1 : 10 : 0,5 bedeutet. Die Bildung der Heteropolysäure verläuft besonders günstig in der Siedehitze unter Normaldruck und ist an einer allmählichen Rot-Orange-Färbung der wäßrigen Lösung zu erkennen. Im geschlossenen Gefäß kann die Umsetzung auch bei höheren Temperaturen, d.h. z.B. Gebiet von 100 bis 250° C durchgeführt werden, wodurch die Reaktionszeit deutlich verkürzt werden kann. Bei der Herstellung kann die Konzentration der Heteropolysäure in der wäßrigen Lösung eben weiten Bereich, z.B. 10 bis 50 Gew.-% einnehmen. Durch Aufkonzentrieren der wäßrigen Lösung wird die Verbindung durch Kristallisation gewonnen.

b) Herstellung der Katalysatoren

Die Verwendung der $H_8PMo_{10}VO_{39} \cdot 32\ H_2O$- bzw. der mehr oder weniger kristallwasserfreien Heteropolysäure oder auch des durch Tempern der Heteropolysäure bis auf Temperaturen um 400° C erhaltbaren Anhydrids der Formel $PMo_{10}VO_{35}$ zur Herstellung von Katalysatoren kann nach Angaben geschehen, wie sie in der DE-OS 32 48 600, Seiten 5 u. 6, für die Herstellung von Cu-haltigen Mo-V-P-Heteropolysäurekatalysatoren beschrieben sind. Danach kann die neue Heteropolysäure, insbesondere nach Zusatz inerter Stoffe, zu Granulat-Katalysatoren, zu Schalenkatalysatoren, zu Imprägnier- oder Tränkkatalysatoren und zu Katalysatorpreßlingen weiterverarbeitet wenden.

c) Oxidehydrierungsverfahren

Erfindungsgemäß wird der so erhaltene Katalysator zur oxidativen Dehydrierung von Isobuttersäure zu Methacrylsäure oder der entsprechenden niederen Alkylester, wie Methylisobutyrat, zu entsprechenden niederen Alkylestern der Methacrylsäure, wie Methylmethacrylat, in der Dampfphase bei Temperaturen von 250 bis 400° C, vorzugsweise 300 bis 380° C, eingesetzt. Das Einsatzgasgemisch enthält pro Mol Isobuttersäure bzw. ihrem Ester 1 bis 5 Mol Sauerstoff, im allgemeinen in Form von Luft, 0 bis 8 Mol, vorzugsweise 0 bis 4 Mol, Wasserdampf und gegebenenfalls weitere, inerte Verdünnungsgase wie $N_2$, CO, $CO_2$, die als Kreisgasgemisch aus der Oxidehydrierung stammen können und mit dem eingebrachten Luftstickstoff bei 10 bis 20 Mol weiterem Inertgas liegen.

Das Verfahren kann im Kreislauf- oder Rohrreaktor, auch in mehreren gegebenenfalls hintereinandergeschalteten, Reaktionssystemen durchgeführt werden, wobei z.B. für die Vorumsetzung ein Kreislaufreaktor und für die Endumsetzung ein Rohrreaktor verwendet werden. Der Katalysator kann im Dauerbetrieb mit etwa (1 bis 20)$\cdot 10^{-2}$ Mol Isobuttersäure oder deren Ester je kg Katalysatorfüllung und Minute belastet werden. Man erreicht im Kreislaufreaktor Umsätze bis in den Bereich von 80 bis 90 % der eingesetzten Isobuttersäure(ester), im Rohrreaktor bis nahe an 100%.

Beispiele

Beispiel 1

Herstellung von Dekamolybdovanadophosphorsäure ($H_8PMo_{10}VO_{39} . 32H_2O$)

In eine Lösung von 98 g (1 Mol) $H_3PO_4$ in 7550 g entionisiertem Wasser wurden 1439,5 g (10 Mol) $MoO_3$ und 90,95 g (0,5 Mol) $V_2O_5$ suspendiert und anschließend dieses Gemisch 18 Stunden unter Rühren bei 100° C erhitzt. Nach 6 Stunden hatte sich praktisch schon alles zu einer orange-roten Lösung aufgelöst. Die abgekühlte Lösung wurde von einer Spur Ungelöstem durch Filtration befreit und anschließend die ca. 18 %ige wäßrige Heteropolysäurelösung am Rotationsverdampfer auf praktisch 50 % Heteropolysäuregehalt aufkonzentriert.

Ein Teil dieser Lösung wurde zur Kristallisation gebracht und die orangefarbenen Kristalle zweimal aus Wasser umkristallisiert. Durch Tempern auf 120° C und auf 150° C wurde aus den Gewichtsverlusten der Substanz ein Wassergehalt von 25,6 Gew.-% ermittelt, der 32 Mol Kristallwasser pro Mol, nach Mo- und V-Bestimmung als $H_8PMo_{10}VO_{39}$ zusammengesetzten, Heteropolysäure entspricht. Das mit Cu-Kα-Strahlung

erhaltene Röntgendiagramm der kristallwasserhaltigen Verbindung, ist demjenigen der $H_5PMo_{10}V_2O_{40}$ . (30-35) $H_2O$ sehr ähnlich. Die kristallwasserfreien Verbindungen liefern im Vergleich dagegen Röntgendiagramme mit z.T. deutlich verschiedenen 2 $\theta$-Werten.

$H_8PMo_{10}VO_{39}$(1673,49) Ber. Mo 57,34 % Gef. Mo 57,6 %
V 3,05 % V 3,06 %

$^{31}P$-NMR: $\delta$ = 3,35 ppm; gemessen als ca. 50 % wäßrige Lösung bei 80,98 MHz; $H_3PO_4$ als externen Standard.

Beispiel 2

Herstellung von $PMo_{10}VO_{35}$

100 g der kristallwasserfreien $H_8PMo_{10}VO_{39}$-Verbindung wurden durch Erhitzen von 200 a 300° C bis zur Gewichtskonstanz getempert. Dabei trat ein Gewichtsverlust von 4,28 g ein, der 4 Mol Wasser der obigen Verbindung entspricht. Die Mo- und V-Bestimmung des ockerfarbenen Rückstandes zeigte, daß eine Verbindung $PMo_{10}VO_{35}$ vorlag und die Heteropolysäure durch Abgabe ihres Konstitutionswassers in ein Anhydrid übergegangen war.

$PMo_{10}VO_{35}$(1601,43) Ber.Mo 59,92 % Gef. Mo 59,9 %
V 3,18 % V 3,2 %

Beispiel 3

Granulat-Katalysator aus 70 % $H_8PMo_{10}VO_{39}$ und 30 % $SiO_2$

Zu 100 g der 50 %igen $H_8PMo_{10}VO_{39}$-Lösung wurden 18,0 g Kieselgur und 3,5 g Aerosil® 200 pulvrig zugesetzt und die ganze Mischung zur Trockne eingedampft und dann 6 Stunden bei 120° C getrocknet. Danach wurde die Masse zu ca. 5 mm großen orangefarbenen Granulatstücken zerkleinert. Vor dem Einsatz als Katalysator wurden diese noch 3 Stunden bei 300° C calciniert.

Oxidative Dehydrieruug von Isobuttersäure zu Methacrylsäure

Beispiel 4

Katalyse im Kreislaufreaktor mit Katalysator nach Beispiel 3

Über 107,1 g, entsprechend 130 ml, des nach Beispiel 3 hergestellten Katalysators wurde pro Stunde ein dampfförmiges Gemisch aus 67,4 g Isobuttersäure, 133 Liter Luft (gemessen bei 20° C) und 262 Liter Stickstoff (gemessen bei 20° C), was einem Molverhältnis Isobuttersäure : Sauerstoff : Stickstoff = 1 : 1,5 : 20 entspricht, geleitet. Die Reaktionstemperatur war im Bereich von 324 bis 330° C. Nachfolgend die erhaltenen Ergebnisse nach zwei verschiedenen Zeiten:

| Reaktionszeit (Stunden) | Umsatz Isobuttersäure (%) | Selektivität Methacrylsäure (%) | Raum-Zeit-Ausbeute Methacrylsäure ($g \cdot l^{-1} \cdot h^{-1}$) |
|---|---|---|---|
| 27,5 | 80,2 | 74,2 | 301,3 |
| 50 | 79,8 | 75,1 | 303,3 |

Beispiel 5

In einem Kreislaufreaktor wurde über 107 g, entsprechend 130 ml, 3 Stunden bei 300° C calciniertem, 2 bis 5 mm großem Granulat, hergestellt aus $H_8PMo_{10}VO_{39}$, enthaltend 70 % $PMo_{10}VO_{35}$ und 30 % $SiO_2$ - (Kieselgur und Aerosil® 200 im Verhältnis 5 : 1) pro Stunde ein dampfförmiges Gemisch aus 67,4 g Isobuttersäure, 266 Liter Luft (gemessen bei 20° C) und 130 Liter Stickstoff (gemessen bei 20° C), was einem Molverhältnis Isobuttersäure : Sauerstoff : Stickstoff von 1 : 3 : 18,5 entspricht, geleitet. Die Reaktionstemperatur war 325 bis 330° C. Der 426 Stunden laufende Versuch zeigte, daß Aktivität und Selektivität über die ganze Versuchsdauer konstant blieben. Die unter den angegebenen Bedingungen erzielten Isobuttersäureumsätze lagen praktisch konstant bei 82 bis 83 % mit Methacrylsäureselektivitäten um 72 %.

Beispiele 6 und 7 und Vergleichsbeispiele 1 und 2

Vergleich von 3 Stunden bei 400° C calcinierten aus $H_8PMo_{10}VO_{39}$ bzw. $H_5PMo_{10}V_2O_{40}$ hergestellten Katalysatoren, enthaltend je 30 % $SiO_2$ (Kieselgur-Aerosil® 200 = 5 : 1), Prüfungen im Rohrreaktor.

Bei Verweilzeiten von 0,3 sec bei 350° C Reaktionstemperatur wurde über je 2 ml Katalysator (Partikelgröße 0,75 bis 1 mm) ein dampfförmiges Gemisch aus Isobuttersäure, Wasser und Sauerstoff (als Luft) im Molverhältnis 1 : 2 : 1,5 geleitet. Thermische Vorbehandlung der Katalysatoren: Nach 3-stündigem Trocknen bei 200° C im Trockenofen in Luftatmosphäre wurden die Proben in den Glasreaktoren 3 Stunden im Salzbad bei 400° c in einem Luft- oder Stickstoffstrom (je 6 l Luft bzw. Stickstoff pro Stunde) calciniert. Aus der folgenden Zusammenstellung sind die erhaltenen Ergebnisse in Abhängigkeit von der Zusammensetzung des katalytischen Materials und der Vorbehandlung des Katalysators zu ersehen.

| Beisp. Nr. | Katalysator aus | Calcinieratmosphäre | Umsatz Isobuttersäure | Selektivität Methacrylsäure |
|---|---|---|---|---|
| 6 | $H_8PMo_{10}VO_{39}$ | Stickstoff | 97,5 | 63,3 |
| 7 | $H_8PMo_{10}VO_{39}$ | Luft | 95,9 | 61,6 |
| **Vergleichsbeispiel 1** | | | | |
| | $H_5PMo_{10}V_2O_{40}$ | Stickstoff | 75,8 | 48,4 |

Vergleichsbeispiel 2

| | | | |
|---|---|---|---|
| $H_5PMo_{10}V_2O_{40}$ | Luft | 63,1 | 34,2 |

Beide Katalysatoren bei 300°C getempert, bringen unter sonst gleichen Prüfbedingungen Isobuttersäureumsätze von 95 % mit Methacrylsäureselektivitäten von 65 bis 70 %.

Beispiel 8 und Vergleichsbeispiel 3

Bei 330°C wurde in einen Kreislaufreaktor über 130 ml Katalysator ein Isobuttersäure-Sauerstoff-Stickstoffgemisch vom Molverhältnis 1 : 1,5 : 20 mit einer Verweilzeit von 0,6 sec geleitet.
Aus dem Vergleich der Ergebnisse des folgenden Beispiels 8 mit dem Vergleichsbeispiel 3 ist der relativ schnelle Aktivitätsverlust des in der DE-OS 32 48 600 beschriebenen und sehr selektiven $Cu_{0,2}PMo_{10}VO_{35,2}$-Katalysators gegenüber einem erfindungsgemäßen Katalysator ersichtlich.

| Beisp. Nr. | Kat. mit 30 % $SiO_2$ aus | Zeit (h) | Umsatz Isobuttersäure (%) | Selektivität Methacrylsäure (%) |
|---|---|---|---|---|
| 8 | $H_8PMo_{10}VO_{39}$ | 30 | 80,1 | 73,8 |
| | | 70 | 79,7 | 73,8 |
| | | 184 | 79,9 | 73,7 |
| **Vergleichsbeispiel 3** | | | | |
| | $Cu_{0,2}PMo_{10}VO_{35,2}$ | 15 | 79,6 | 79,8 |
| | | 70 | 72,8 | 79,1 |
| | | 140 | 60 | 79,1 |

Beispiel 9

Kugelförmige Katalysatorträger von 8 mm Durmesser, im wesentlichen bestehend aus 93,1 % $Al_2O_3$ und 5,6 % $SiO_2$, mit einer inneren Oberfläche von 0,3 bis 0,37 $m^2/g$ und einer scheinbaren Porosität von 55 bis 60 %, wurden in einer ca. 70 %igen wäßrigen $H_8PM_{10}VO_{39}$-Lösung auf 100°C erhitzt, die Mischung anschließend langsam abkühlen lassen und dann von der überschüssigen Lösung abfiltriert. Die mit katalytischem Material getränkten Kugeln wurden dann 1 Stunde bei 110°C und anschließend 3 Stunden bei 150°C getrocknet. Der Gehalt des Katalysators an $H_8PMo_{10}VO_{39}$ war 33,7 %.
Über 169,9 g (130 ml) des orangefarbenen Kugel-Katalysators in eins Kreislaufreaktor wurden bei 340°C ein dampfförmiges Gemisch aus Isobuttersäure und Sauerstoff (als Luft) im Molverhältnis 1 : 1,5 mit einer Belastung der reinen Katalysatormasse von 0,0002364 Mol Isobuttersäure pro Gramm katalytischer Masse und pro Minute geleitet. Unter diesen Bedingungen wurden ein Isobuttersäureumsatz von 70,2 % und eine Methacrylsäureselektivität von 76 % erhalten. Die Raum-Zeit-Ausbeute an Methacrylsäure betrug 287 g/l•h.

Beispiel 10

Kugelförmige Katalysatorträger von 5 mm Durchmesser, im wesentlichen bestehend aus 65,8 % SiC, 28,5 % $SiO_2$ und 4,7 % $Al_2O_3$, mit einer inneren Oberfläche von 0,01 bis 0,3 $m^2/g$ und einer scheinbaren Porosität von 43 bis 48 %, wurden wie in Beispiel 9 beschrieben mit $H_8PMo_{10}VO_{39}$ imprägniert. Der Gehalt des getrockneten Katalysators an $H_8PMo_{10}VO_{39}$ betrug 27,2 %. Über 166,8 g (130 ml) des orange-braunen Katalysators in einem Kreislaufreaktor wurde bei 320 °C ein dampfförmiges Gemisch aus Isobuttersäure und Sauerstoff (als Luft) im Molverhältnis 1 : 1,5 mit einer Belastung der reinen Katalysatormasse von 0,0004729 Mol Isobuttersäure pro Gramm katalytischer Masse und pro Minute geleitet. Es wurde ein Isobuttersäureumsatz von 79,1 %, eine Methacrylsäureselektivität von 70 % und eine RZA von 470 g Methacrylsäure/l•h ermittelt.

## Patentansprüche

1. Heteropolysäure mit den Atomen Phosphor, Molybdän und Vanadium der Zusammensetzung $H_8PMo_{10}VO_{39}$.

2. Anhydrid der Heteropolysäure nach Anspruch 1 mit der Zusammensetzung $PMo_{10}VO_{35}$.

3. Verfahren zur Herstellung der Heteropolysäure $H_8PMo_{10}VO_{39}$,

   dadurch gekennzeichnet,

   daß man eine wäßrige Aufschlämmung der Oxide bzw. Oxisäuren von Molybdän, Vanadium und Phosphor in dem für die Bildung der $H_8PMo_{10}VO_{39}$ stöchiometrischem Verhältnis, hydrothermisch im Temperaturbereich von 60 bis 250 °C und einem Druck zwischen Normaldruck und dem sich im geschlossenen Gefäß einstellenden, von der Temperatur abhängigen Wasserdampfdruck, zu einer wäßrigen Lösung dieser Säure umsetzt, und die Heteropolysäure daraus in kristallwasserhaltiger Form isoliert.

4. Verfahren zur Herstellung der kristallwasserfreien $H_8PMo_{10}VO_{39}$, dadurch gekennzeichnet, daß die nach Anspruch 3 erhaltene kristallwasserhaltige Heteropolysäure auf Temperaturen von 100 bis 200 °C erhitzt wird.

5. Verfahren zur Herstellung des Heteropolysäureanhydrids $PMo_{10}VO_{35}$ aus $H_8PMo_{10}VO_{39}$ oder der entsprechenden kristallwasserhaltigen Heteropolysäure, dadurch gekennzeichnet, daß die Heteropolysäure auf Temperaturen über 200 bis 400 °C erhitzt wird.

6. Verwendung von $H_8PMo_{10}VO_{39}$, gegebenenfalls im Gemisch mit Trägerstoffen, als Katalysator in säurekatalysierten Reaktionen zur Herstellung organischer Verbindungen.

7. Verwendung von $H_8PMo_{10}VO_{39}$ und des daraus bei höherer Temperatur gebildeten $PMo_{10}VO_{35}$, gegebenenfalls im Gemisch mit Trägerstoffen, als Katalysatoren in Oxidationsreaktionen zur Herstellung organischer Verbindungen.

8. Verwendung von $H_8PMo_{10}VO_{39}$ und des daraus bei höherer Temperatur gebildeten $PMo_{10}VO_{35}$ als Katalysatoren nach Anspruch 7, für die Oxidehydrierung von Isobuttersäure oder ihrer niederen Ester in der Dampfphase in Gegenwart von Sauerstoff bei einer Temperatur über 300 °C zur Herstellung von Methacrylsäure oder ihren niederen Estern.

## Claims

1. Heteropolyacid with the atoms phosphorus, molybdenum and vanadium having the composition $H_8PMo_{10}VO_{39}$.

2. Anhydride of the heteropolyacid according to claim 1 having the composition $PMo_{10}VO_{35}$.

3. Process for preparing the heteropolyacid $H_8PMo_{10}VO_{39}$, characterised in that an aqueous suspension of the oxides or oxiacids of molybdenum, vanadium and phosphorus in the stoichiometric ratio for forming $H_8PMo_{10}VO_{39}$ is hydrothermically reacted, in a temperature range from 60 to 250 °C and

under a pressure between normal pressure and the water vapour pressure produced in the sealed vessel and dependent on the temperature, to yield an aqueous solution of this acid, and the heteropolyacid is isolated therefrom in a form which contains water of crystallisation.

**4.** Process for preparing $H_8PMo_{10}VO_{39}$ free from water of crystallisation, characterised in that the heteropolyacid containing water of crystallisation and obtained according to claim 3 is heated to temperatures of 100 to 200 °C.

**5.** Process for preparing the heteropolyacid anhydride $PMo_{10}VO_{35}$ from $H_8PMo_{10}VO_{39}$ or the corresponding heteropolyacid which contains water of crystallisation, characterised in that the heteropolyacid is heated to temperatures above 200 to 400 °C.

**6.** Use of $H_8PMo_{10}VO_{39}$, optionally in admixture with carrier substances, as a catalyst in acid-catalysed reactions for producing organic compounds.

**7.** Use of $H_8PMo_{10}VO_{39}$ and the $PMo_{10}VO_{35}$ formed therefrom at higher temperature, optionally in admixture with carrier substances, as catalysts in reactions of oxidation to produce organic compounds.

**8.** Use of $H_8PMo_{10}VO_{39}$ and the $PMo_{10}VO_{35}$ formed therefrom at elevated temperature as catalysts according to claim 7, for the oxidehydration of isobutyric acid or the lower esters thereof in the vapour phase in the presence of oxygen at a temperature of above 300 °C for producing methacrylic acid or the lower esters thereof.

**Revendications**

**1.** Hétéropolyacide renfermant les atomes phosphore, molybdène et vanadium, ayant la composition $H_8PMo_{10}VO_{39}$.

**2.** Anhydride de l'hétéropolyacide selon la revendication 1, ayant la composition $PMo_{10}VO_{35}$.

**3.** Procédé de préparation de l'hétéropolyacide $H_8PMo_{10}VO_{39}$, caractérisé en ce qu'on transforme hydrothermiquement une suspension aqueuse des oxydes ou oxacides de molybdène, de vanadium et de phosphore, dans le rapport stoechiométrique pour la formation de $H_8PMo_{10}VO_{39}$, en une solution aqueuse de cet acide, dans la gamme de température de 60 à 250 °C et sous une pression comprise entre la pression normale et la pression de vapeur d'eau, dépendante de la température, qui s'établit dans le récipient fermé, et on isole l'hétéropolyacide à partir de cette solution, sous une forme contenant de l'eau de cristallisation.

**4.** Procédé de préparation du $H_8PMo_{10}VO_{39}$ exempt d'eau de cristallisation, caractérisé en ce qu'on chauffe à des températures de 100 à 300 °C l'hétéropolyacide contenant de l'eau de cristallisation qui est obtenu selon la revendication 3.

**5.** Procédé de préparation de l'anhydride d'hétéropolyacide $PMo_{10}VO_{35}$ à partir de $H_8PMo_{10}VO_{39}$ ou de l'hétéropolyacide contenant de l'eau de cristallisation correspondant, caractérisé en ce qu'on chauffe l'hétéropolyacide à des températures de plus de 200 à 400 °C.

**6.** Utilisation de $H_8PMo_{10}VO_{39}$, éventuellement en mélange avec des matières de support, comme catalyseur dans des réactions catalysées par acide pour la préparation de composés organiques.

**7.** Utilisation de $H_8PMo_{10}VO_{39}$ et de $PMo_{10}VO_{35}$ formé à partir de lui à haute temperature, éventuellement en mélange avec des matières de support, comme catalyseurs dans des réactions d'oxydation pour la préparation de composés organiques.

**8.** Utilisation de $H_8PMo_{10}VO_{39}$ et de $PMo_{10}VO_{35}$ formé à partir de lui à haute température, comme catalyseurs selon la revendication 7, pour l'oxydéshydrogénation d'acide isobutyrique ou de ses esters inférieurs en phase vapeur, en présence d'oxygène et à une température supérieure à 300 °C, pour la préparation d'acide méthacrylique ou de ses esters inférieurs.